(19) Europäisches Patentamt
European Patent Office
Office européen des brevets

(11) EP 0 486 437 B1

(12) EUROPEAN PATENT SPECIFICATION

(45) Date of publication and mention of the grant of the patent:
**09.07.1997 Bulletin 1997/28**

(51) Int Cl.[6]: **C08G 63/42**, C08G 64/00, C08G 63/84, C08G 64/34

(21) Application number: **91830485.8**

(22) Date of filing: **11.11.1991**

# (54) Functional polyesters from glycidyl derivatives and cyclic anhydrides or carbon dioxide

Funktionelle Polyester aus Glycidylderivaten und cyclischen Anhydriden oder Kohlendioxid

Polyesters fonctionnels à partir de dérivés glycidyliques et d'anhydrides cycliques ou de dioxyde de carbone

(84) Designated Contracting States:
**AT BE CH DE DK ES FR GB GR IT LI LU NL SE**

(30) Priority: **13.11.1990 IT 1771390**
**13.11.1990 IT 1771490**

(43) Date of publication of application:
**20.05.1992 Bulletin 1992/21**

(73) Proprietor: **ISTITUTO SIEROVACCINOGENO ITALIANO I.S.I. S.p.A.**
**I-55020 Castelvecchio Pascoli, Lucca (IT)**

(72) Inventors:
- **Chiellini, Emo**
  **I-56100 Pisa (IT)**
- **Solaro, Roberto**
  **I-56100 Pisa (IT)**
- **Bemporad, Luca**
  **I-56100 Pisa (IT)**

(74) Representative: **de Simone, Domenico et al**
**Ing. Barzanò & Zanardo Roma S.p.A.**
**Via Piemonte 26**
**00187 Roma (IT)**

(56) References cited:
**BE-A- 689 483**

- **CHEMICAL ABSTRACTS, vol. 82, no. 10, 19th May 1975, page 19, column 2, abstract no. 125861f, Columbus, Ohio, US; & JP-A-74 99 393 (TOYO SODA MFG. CO., LTD) 19-09-1974**
- **CHEMICAL ABSTRACTS, vol. 78, no. 8, 26th February 1973, page 34, column 1, abstract no. 44471u, Columbus, Ohio, US; & SU-A-351 835 (A.L. SHAPIRO et al.) 21-09-1972**
- **DIE MAKROMOLEKULARE CHEMIE, vol. 178, no. 1, January 1977, pages 47-54, Basel, DE; W. KURAN et al.: "Organozinc catalyst systems for the copolymerization of carbon dioxide with propylene oxide"**
- **DIE MAKROMOLEKULARE CHEMIE, vol. 180, no. 9, September 1979, pages 2153-2161, Basel, DE; A. ROKICKI et al.: "Copolymerization of carbon dioxide with propylene oxide in the presence of catalysts based on alkylmetal compounds and pyrogallol"**
- **W. BAILEY et al.: "Contemporary Topics in Polymer Science", 1984, pages 343-360; S. INOUE: "Copolymerization of carbon dioxide and epoxide and related reactions", Plenum Press, New York, US**
- **JOURNAL OF BIOACTIVE AND COMPATIBLE POLYMERS, vol. 5, no. 1, January 1990, pages 16-30, Technomic Publishing Co., Inc.; E. CHIELLINI et al.: "Polyesters based on glyceric acid derivatives as potential biodegradable materials"**
- **DIE MAKROMOLEKULARE CHEMIE, vol. 178, no. 5, May 1977, pages 1283-1294, Basel, DE; H. KOINUMA et al.: "Copolymerization of carbon dioxide and some oxiranes by organoaluminium catalysts"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention deals with the synthesis of high molecular weight polyesters and polycarbonates. In particular it is related to the preparation of new bioerodible polymers containing two carboxyl groups per repeating unit. One of these groups is bound throughout an ester bond to a variety of functional hydrophilic or potentially hydrophilic groups, whereas the other one is linked to hydrophobic moieties. The resulting polyhydroxylated polyesters and polycarbonates are well suited for the production of thermoplastics and thermosets and for the formulation of biodegradable systems to be used in pharmaceutics and biomedicine.

BACKGROUND ART

Polyesters are condensation products characterized by a regular distribution of ester bonds along polymer main chain (Goodman e Rhys, in Polyesters, Vol. I -Saturated polymers, The Plastic Institute, Iliffe Books, London, 1965). Formally, they are produced by esterification of dicarboxylic acids with diols or by self-condensation of hydroxyacids. However, a variety of synthetic procedures to polyesters exists, particularly polyaddition reactions in which monomer molecules react by a chain process.

Direct esterification of dicarboxylic acids with diols and self-condensation of hydroxyacids, either in the presence or in the absence of suitable catalysts, are not generally used for large scale application, due to the high reaction temperature, that can give rise to appreciable decomposition of both starting monomers and produced polymer, and the difficult stoichiometric dosage of reagents. This last point applies to all polycondensation reactions and can limit severely the attainment of high molecular weight product. To overcome this problem, a two steps reaction scheme can be applied, implying the prior formation of a dihydroxy-diester by reaction of the diacid with two equivalents of the diol, followed by a polycondensation reaction in which a diol molecule is eliminated at each condensation step. In order to attain high molecular weights, the by-product formed must be removed from the reactor. However, better results are generally obtained by replacing carboxylic diacids with dialkyl or diaryl esters, that can be more easily purified and have a larger reactivity. Analogously, polycarbonates can be produced by reacting diols with diesters of carbonic acid, whereas hydroxyesters are more convenient than hydroxy acids in the self-condensation reaction. Also in the case of diesters better results are attained by a two step process in which the polymerizable intermediate is again a dihydroxy-diester. Dicarboxylic acids and diols can be conveniently replaced by the corresponding acid dichlorides and dialkyl carbonates, respectively.

Polyesters can be formed also by polyaddition reactions. The most important one is the polymerization of lactones or lactides, that under mild conditions give rise to the same polymeric product in principle attainable by self-condensation of the corresponding hydroxyacid. Other possible routes are based on the alternating copolymerization of cyclic anhydrides with cyclic ethers, such as epoxides and tetrahydrofuran, of dicarboxylic acids with diol divinyl ethers, and of diols with bisketenes. These last can be copolymerized also with aldehydes, ketones, and formates.

Some polyesters, such as poly( ε-caprolactone), poly(orthoester)s and copolymers of glycidic acid with lactic acid have been accepted by competent authorities (as FDA) for applications in biomedicine and in the food industry, due to their biocompatibility and biodegradability to non toxic products. The large amount of reports on the degradation of polyesters and their structural analogues has been reviewed by several authors (Reed e Gilding, Polymer, 22, 494, 1981; Jarret et al., in Polymers as biomaterials, Plenum Press, New York, 1984, p. 181; Heller, J. Controlled Release, 2, 167, 1985; Holland et al., J. Controlled Release, 4, 155, 1987; Pitt e Gu, J. Controlled Release, 4, 283, 1987; St. Pierre e Chiellini, J. Bioact. Compat. Polym., 2, 4, 1987). However, only few examples have been reported on polyfunctional synthetic polyesters that due to the presence of hydrophilic substituents are water soluble and therefore well suited to undergo chemical and biochemical degradation processes (Vert e Lenz, U.S. Pat. 4.265.247, Lug. 1981; Braud et al., Polym. Bull., 13,293, 1985, Chiellini et al., J. Bioact. Compat. Polym., 5, 15, 1990).

On the other hand, aliphatic polycarbonates have not received much attention, due to their rather low mechanical properties (Carothers e Natta, J. Am. Chem. Soc., 52, 314, 1930). and only the reaction product of allyl alcohol with diethylen chloroformate has received some interest (Watanabe, The chemistry of commercial plastics, Reinhold Pub. Co., New York, 1947).

Even though the production of poly(alkyl carbonates) by catalyzed reaction of diols with carbon monoxide is known (US Pat. 4.131.152), the only practical pathway to aliphatic polycarbonates is the method based on the reaction of epoxides with carbon dioxide in the presence of suitable catalysts (Contemporary Topcs in Polymer Science, W.J. Bailey, T. Tsuruta, Eds., Plenum Press, New York, 1984, p. 343). At the present, none of the above materials has found applications in biomedicine or pharmaceutics, mainly due to their hydrophobicity. However, functional polycarbonates should have superior properties for biomedical use. Moreover, even if several polymers have been investigated for the controlled release of drugs, the results obtained are not satisfactory and require for the use of new materials. In this respect, the polycarbonates that are the subject of the present invention appear to be useful due to their large and modulable hydrophilicity and also because they give rise only to natural biodegradation products, such as carbon

dioxide and polyols, that are not toxic and can be easily degraded by the human body.

DISCLOSURE OF INVENTION

The present invention describes new biodegradable functional polyesters and polycarbonates having the following formula:

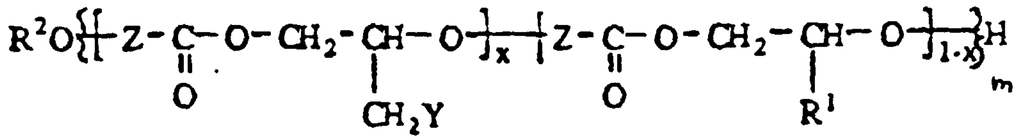

where:

- R1 is either H, or alkyl, or 1-(2-pyrrolidonyl) alkoxymethyl, or 1-(2- pyrrolidonyl)alkyl;
- R2 is either H, or alkyl, or aryl;
- Z is either -CO-R3- residue where R3 is linear or branched alkanediyl or arylalkanediyl or arylenediyl, or is substituted by a carbon-oxygen bond;
- Y is either glyceryl, or pentitolyl, or heptitolyl;
- $0 < x \leq 1$; $5 < m < 500$.

to be used as thermoplastic and thermoset materials and as biodegradable matrices in the controlled release of active principles of biomedical, pharmaceutical and agricultural interest. Due to the presence in the side chain of vicinal hydroxyl groups, their use as heavy metal sequestrants is also possible.

According to the invention, a process of production is described by copolymerization of the glycidyl ethers of alditols (having preferably but not exclusively three, five, and seven carbon atoms) protected with isopropylidene groups and their mixtures with alkylene oxides (preferably but not exclusively ethylene oxide and propylene oxide) with either cyclic anhydrided (at 30-150C) or with carbon dioxide (at 30-60C) in the presence of different catalysts, preferably but not exclusively alkylaluminum derivatives in the first case and dialkylzinc/polyhydroxyphenols systems in the second one.

The hydrophilicity of the polymeric materials thus obtained, having molecular weight included between 1000 and 100.000, preferably between 2.000 and 30,000, can be modulated by controlled removal, either partial or total, of isopropylidene protecting groups. This reaction is promoted by low and high molecular weight acid catalysts and does not appreciably affect the degree of polymerization of the reacted polymers.

Removal of the isopropylidene protecting groups can be carried out by precipitation of the protected polyesters and polycarbonates in methanol containing 5% of concentrated HCl. Alternatively the deprotection reaction can be performed by heating a polymer solution in methanol/chloroform mixtures in the presence of a styrene/divinylbenzene sulphonic resin.

Accordingly, it is possible to attain a large number of polyesters and polycarbonates characterized by a different content of hydroxyl groups in the side chains and having a molecular weight included between 800 and 80,000, preferably between 2.000-25.000.

DETAILED DESCRIPTION OF THE INVENTION

In the following examples detailed description of the preparation of the polyesters of the invention are provided, including also the preparation of glycidyl derivatives.

<u>Preparation of Glycidic Monomers</u>

Example 1 <u>1-O-Glycidyl-2,3-O-isopropylideneglycerol GIG</u>

A suspension of 11.0 g (0,46 mol) of NaH in 600 ml of anhydrous THF was placed under dry nitrogen in a 3-necked 1000 ml flask, equipped with condenser, dropping funnel and mechanical stirrer. Then 50 ml (0.38 mol) of (R),(S)-isopropylideneglycerol (Solketal) were added over 1.5 h under stirring and then refluxed for 3 h. 400 ml (25 mol) of the resulting suspension was added under dry nitrogen over 3 h to 70 ml (0.90 mol) of epichlorohydrin (EPY) in a 3-necked 1000 ml flask, equipped with condenser, dropping funnel and mechanical stirrer. The mixture was stirred overnight at room temperature and then refluxed for 6 h. After cooling, the solid was filtered off. The resulting solution, after removal

of the volatile products, was distilled under vacuum over calcium hydride to yield 33.6 g (71%) of a colourless liquid having b.p. 76°C/0.2 mm that was characterized by IR and NMR spectroscopy.

IR (liquid film): $\bar{\nu}$ = 3040 ($\nu$ CH epoxide), 3000.2800 ($\nu$ CH aliphatic), 1456 ($\delta$ $CH_2$ and $\delta_{as}$ $CH_3$), 1380 ($\delta$ $CH_3$), 1250-1040 ($\nu$ C-O-C epoxide ed ether), 904 ($\nu$ C-O-C epoxide) and 844 $cm^{-1}$ ($\nu$ epoxide and dioxolane ring).

$^1$H-NMR ($CDCl_3$): $\delta$ = 1.3 and 1.4 (two s, 6H, $CH_3$), 2.6 (m, 1H, CH-9a), 2.8 (dd, 1H, CH-9b), 3.2 (m, 1H, CH-8), 3.4-3.9 (m, 5H. $CH_2$ and CH-3a), 4.1 (d.d., 1H, CH-3b) and 4.3 ppm (m, 1H, CH-2).

$^{13}$C-NMR ($CDCl_3$): $\delta$ = 24.87 (C-5), 26.23 (C-6), 43.53 (C-9), 50.23 (C-8), 66.21 (C-7), 71.82, 71.94 and 72.06 (C-1 + C-3), 74.29 and 74.37 (C-2) and 109.11 ppm (C-4).

Example 2 1-0-Glycidyl-2,3-0-isopropylideneglycerol GIG

A solution of 30.0 g (227 mmol) of solketal and 7.4 g (2.3 mmol) of tetrabutylammonium bromide (TBAB) in 90 ml of toluene was added to 100 ml of 50% NaOH water solution in a 3-necked 500 ml flask, equipped with condenser, dropping funnel and mechanical stirrer. The mixture was stirred at room temperature for 1 h, then 35 ml (0.448 mol) of EPY were added under stirring. The resulting suspension was stirred for additional 26 h, then the organic layer was dried over MgSO4. After removal of the volatile products, the residue was distilled under vacuum over calcium hydride to yield 34.3 g (80%) of colourless liquid having b.p. 76°C/0.2 mm that was characterized by IR and NMR spectroscopy.

Example 3 0-Glycidyl-di-0-isopropylidenexylitol XPG

A suspension of 110 g of $ZnCl_2$ in 1.0 l of acetone was stirred for 3 h at room temperature and then left standing overnight. The surnatant was transferred under dry nitrogen into a 2000 ml two-necked flask equipped with mechanical stirrer, then 80.0 g (0.53 mol) of xylitol were added and the reaction mixture was kept under stirring for 17 h. The resulting solution was poured into a 2000 ml flask and 2.01 of 20% NaOH water solution were slowly added. The organic layer was evaporated under vaccum, the residue was dissolved in diethyl ether and filtered. After removal of the volatile products, the oily residue was distilled under vacuum to yield 95.1 g (78%) of a viscous liquid having b.p. 108-113°C/0.2 mm and constituted by a 12:1:8 mixture of three products, as shown by g.l.c. analysis. The mixture was characterized also by IR and NMR spectroscopy. A solution of 77.0 g (0.332 mol) of di-0-isopropylidenexylitol and 10.6 g (33 mmol) of tetrabutylammonium bromide in 200 ml of toluene was added to 250 ml of a 50% NaOH water solution in a 1 l three-necked flask equipped with condenser, dropping funnel and mechanical stirrer. The mixture was stirred at room temperature for 1 h, then 55 ml (0.70 mol) of EPY were added under stirring. The resulting suspension was stirred for additional 28 h, then the organic layer was dried over $Na_2SO_4$. After removal of the volatile products, the residue was distilled under vacuum over calcium hydride to yield 83.7 g (87%) of colourless liquid having b.p. 150-153°C/0.5 mm and constituted by a 2:1 mixture of two isomers, as shown by g.l.c. analysis. The mixture was characterized also by IR and NMR spectroscopy.

IR (liquid film): $\bar{\nu}$ = 3052 ($\nu$ CH epoxide), 3000-2800 ($\nu$ CH aliphatic),1456 ($\delta$ $CH_2$ and $\delta_{as}$ $CH_3$), 1380 ($\delta_s$ $CH_3$), 1250-1040 ($\nu$ C-O-C), 904 ($\nu$ C-O-C epoxide) and 844 $cm^{-1}$ (epoxide and dioxolane ring).

$^1$H-NMR ($CDCl_3$): $\delta$ = 1.2-1.4 (m, 12H, $CH_3$), 2.5 and 2.7 (two m, 2H, $CH_2$ epoxide), 3.1 (m, 1H, CH epoxide) and 3.3-4.2 ppm (m, 7H, $CH_2$ and CH).

$^{13}$C-NMR ($CDCl_3$): $\delta$ = 25-27 ($CH_3$), 43.5-44.5 (CH epoxide), 50-51 ($CH_2$ epoxide), 65-81 (CH-O + $CH_2$-O) and 109-110 ppm (O-C-O).

Example 4 L-O-Glycidyl-di-O-isopropylidenearabitol APG

A suspension of 50 g of $ZnCl_2$ in 400 ml of acetone was stirred for 3 h at room temperature and then left standing overnight. The surnatant was transferred under dry nitrogen into a 2000 ml two-necked flask equipped with mechanical stirrer, then 25.0 g (0.164 mol) of L-arabitol were added and the reaction mixture was kept under stirring for 17 h. The resulting solution was poured into a 2000 ml flask and 800 ml of a 20% NaOH water solution were slowly added. The organic layer was evaporated under vacuum, the residue was dissolved in diethyl ether and filtered. After removal of the volatile products, the oily residue was distilled under vacuum to yield 23.6 g (69%) of a viscous liquid having b.p. 92-94°C/0.1 mm and constituted by a 20:10:1 mixture of three isomers, as shown by g.l.c. analysis. The mixture was characterized also by IR and NMR spectroscopy. A solution of 23.0 g (99 mmol) of L-di-O-isopropylidenearabitol and 3.2 g (9.9 mmol) of tetrabutylammonium bromide in 60 ml of toluene was added to 110 ml of a 50% NaOH water solution in a 500 ml three-necked flask equipped with condenser, dropping funnel and mechanical stirrer. The mixture was stirred at room temperature for 1 h, then 18.5 ml (0.20 mol) of EPY were added under stirring. The resulting suspension was stirred for additional 42 h, then the organic layer was dried over $Na_2SO_4$. After removal of the volatile products, the residue was distilled under vacuum over calcium hydride to yield 23.0 g (81%) of colourless liquid having b.p. 110-115°C/0.1 mm that gave only one broad peak at the g.l.c. analysis. The mixture was characterized by IR and NMR

spectroscopy.

IR (liquid film): $\bar{\nu}$ = 3052 ($\nu$ CH epoxide), 3000-2800 ($\nu$ CH aliphatic), 1458 ($\delta$ $CH_2$ and $\delta_{as}$ $CH_3$), 1376 ($\delta_s$ $CH_3$), 1250-1040 ($\nu$ C-O-C), 910 ($\nu$ C-O-C epoxide) and 848 $cm^{-1}$ ($\nu$ epoxide and dioxolane rings).

$^1H$-NMR ($CDCl_3$): $\delta$ = 1.2-1.4 (m, 12H, $CH_3$), 2.5 and 2.7 (two m, 2H, $CH_2$ epoxide), 3.1 (m, 1H, CH epoxide) and 3.3-4.1 ppm (m, 7H, $CH_2$ and CH).

$^{13}C$-NMR ($CDCl_3$): $\delta$ = 25-27 ($CH_3$), 43.5-44.5 (CH epoxide),50-51 ($CH_2$ epoxide), 65-82 (CH-O + $CH_2$-O) and 108.5-110 ppm (O-C-O).

Example 5 N-Glycidylpyrrolidone NGP

A suspension of 21.1 g (0.54 mol) of potassium in 150 ml of anhydrous benzene was placed under dry nitrogen atmosphere in a 500 ml three-necked flask equipped with condenser, dropping funnel and mechanical stirrer, then a solution of 50.6 g (0.58 mol) of 2-pyrrolidone in 110 ml of anhydrous benzene was slowly added under vigorous stirring. The mixture was kept under stirring at room temperature for 3 days and at 40°C one day. The solid product was then filtered, repeatedly washed with anhydrous benzene, and dried under vaccum to yield 65.0 g (98%) of a white hygroscopic powder. A suspension of 69.0 g (.53 mol) of the potassium salt of pyrrolidone in 300 ml of anhydrous THF was slowly added under stirring to 124.0 g (1.35 mol) of epichlorohydrine placed in a 500 ml three-necked flask equipped with condenser and dropping funnel, under dry nitrogen atmosphere. The reaction mixture was heated at reflux for 6 h and then left overnight at room temperature. The solid residue was filtered off, washed with THF and the organic fractions, after removal of the volatile products, were distilled under vacuum to yield 38.5 g (52%) of a colourless liquid having b.p. 97-100°C/0.3 mm. The product was stirred for 3 days at room temperature over $CaH_2$. then distilled again (p.b. 95-96°C/0.2 mm) and analyzed by IR and NMR spectroscopy.

IR (liquid film):$\bar{\nu}$ = 3050 ($\nu$ CH epoxide), 3000-2850 ($\nu$ CH aliphatic), 1680 ($\nu$ C=O), 1500-1400 ($\delta$ $CH_2$ and $\nu$ lactame ring), 1280 ($\nu$ C-O-C and $\nu$ lactame ring), 850 $cm^{-1}$ (epoxide ring).

$^1H$-NMR ($CDCl_3$): $\delta$ = 2.0 (m, 2H, $CH_2$-5), 2.4 (t, 2H, $CH_2$-6), 2.5 (two d, 1H, CH-1b), 2.7 (m, 1H, CH-1a), 3.1 (m, 2H, CH-2c + CH-3e), 3.5 (m, 2H, $CH_2$-4) and 3.8 ppm (two d, 1H, CH-3d).

$^{13}C$-NMR ($CDCl_3$): $\delta$ = 18.1 (C-5), 30.7 (C-6), 44.7 (C-1), 44.8 (C-4), 48-4 (C-3), 50.2, (C-2) and 175.6 ppm (C-7).

Preparation of polymerization catalyst

Example 6 Mesotetraphenylporfinealuminum chloride/tetrabutylammonium bromide TTPAC/TBAB

In a 3000 ml three necked flask equipped with condenser and two dropping funnel, propanoic acid (2 l) was heated at reflux then 54.1 g (800 mmol) of pyrrole and 83.5 g (800 mmol) of benzaldehyde were added at the same time under dry nitrogen atmosphere. After 30 min, the mixture was cooled down to room temperature and filtered. The solid was washed with methanol, hot water and finally dried under vacuum to yield 25.0 g (20%) of violet crystals A solution of 1.23 g (2.0 mmol) of mesotetraphenylporfine in 40 ml of anhydrous dichloromethane was placed under dry nitrogen atmosphere in a 100 ml three necked flask equipped with condenser, dropping funnel and magnetic stirrer, then a solution of 0.29 g (2.4 mmol) of diethylaluminum chloride in 5 ml of anhydrous dichloromethane was slowly added under vigorous stirring. The stirring was continued for 2 h at room temperature, then the solvent was removed and the blue-green solid residue was dissolved in 40 ml of anhydrous dichloromethane. Finally, 0.16 g (0.5 mmol) of tetrabutylammonium bromide was added under vigorous stirring to 10 ml of this solution (corresponding to 0.5 mmol of mesotetraphenylporfinealuminum chloride). The resulting solution was used as a polymerization catalyst immediately after the preparation.

Example 7 Diisobutylzinc/pyrogallol DBZ/Pg

A solution of 1.61 g (9.0 mmol) of DBZ in 20 ml anhydrous dioxane was placed under dry nitrogen atmosphere in a 50 ml three necked flask equipped with condenser, dropping funnel and magnetic stirrer, then a solution of 0.55 g (4.4 mmol) of pyrogallol in 15 ml of dioxane was slowly added and the mixture was kept under stirring at 45°C for 2 h. The resulting clear solution was used as a polymerization catalyst immediately after the preparation.

Example 8 Amberlyst A27 resin, Br- form A27Br

Commercial Amberlyst A27 resin (strong anionic type ion exchange resin, exchange capacity 2.6 meq/g) was left for 36 h under slow stirring in 10% sodium bromide aqueous solution. Every 12 h the bromide solution was replaced by a fresh one, then the resin was filtered, washed with water/ethanol mixtures containing 0,50,70,95 and 100% ethanol and finally dried under vacuum.

Copolymerization of epoxydes with different anhydrides with different catalyst

Example 9 Copolymer of 1-0-glycidyl-2.3-0-isopropyli deneglycerol (GIG) with succinic anhydride.

A solution of 3.0 g (16 mmol) of GIG and 1.6 g (16 mmol) of succinic anhydride in 40 ml of anhydrous toluene containing 0.8 ml (0.6 mmol) of bis(diethylaluminum) sulphate solution was placed under dry nitrogen atmosphere in a 100 ml glass vial. The vial was sealed and then kept at 80°C for 48 h. The resulting solution was filtered and the solvent removed under vacuum to yield 4.5 g (conv. 98%) of polymeric product that was characterized by GPC, IR and NMR analysis. An equimolar amount of both monomeric units was detected in the copolymer by 1H-NMR.

IR (film): $\bar{\nu}$ = 3000-2800 ($\nu$ CH aliphatic), 1740 ($\nu$ C=O), 1458 ($\delta$ $CH_2$ and $\delta_{as}$ $CH_3$), 1374 ($\delta_s$ $CH_3$) and 1300-1000 $cm^{-1}$ ($\nu$ C-O-C).

$^1$H-NMR ($CDCl_3$): $\delta$ = 1.2-1.4 (two s, 6H, $CH_3$), 2.6 (s, 4H, $CH_2$ AS), 3.0-4.5 (m, 9H, CH-O + $CH_2$-O + $CH_2$-OOC) and 5.1 ppm (s, 1H, CH-OOC).

$^{13}$C-NMR ($CDCl_3$): $\delta$ = 25.09 and 26.43 ($CH_3$), 28-29 ($CH_2$ AS), 62-75 (CH-O + $CH_2$-O), 109.40 (O-C-O) and 171.5-174ppm (C=O).

Example 10 Copolymer of 1-0-glycidyl-2.3-0-isopropylideneglycerol with succinic anhydride.

A solution of 3.0 g (16 mmol) of GIG and 1.6 g (16 mmol) of succinic anhydride in 50 ml of anhydrous o-xylene containing 0.8 mmol of TBAB was placed under dry nitrogen atmosphere in a 100 ml glass vial. The vial was sealed and then kept at 120°C for 6 h. The resulting solution was filtered and the solvent removed under vacuum to yield 2.2 g (conv. 48%) of polymeric product that was characterized by GPC, IR and NMR analysis. An equimolar amount of both monomeric units was detected in the copolymer by 1H-NMR.

Example 11 Copolymer of 1-0-glycidyl-2.3-0-isopropylideneglycerol with succinic anhydride.

A solution of 3.0 g (16 mmol) of GIG and 1.6 g (16 mmol) of succinic anhydride in 45 ml of anhydrous o-xylene containing 0.8 mmol of tributylamine was placed under dry nitrogen atmosphere in a 100 ml glass vial. The vial was sealed and then kept at 90°C for 24 h. The resulting solution was filtered and the solvent removed under vacuum to yield 1.5 g (conv. 33%) of polymeric product that was characterized by GPC, IR and NMR analysis. An equimolar amount of both monomeric units was detected in the copolymer by 1H-NMR.

Example 12 Copolymer of 1-0-glycidyl-2.3-0-isopropylideneglycerol with succinic anhydride.

A solution of 3.0 g (16 mmol) of GIG and 1.6 g (16 mmol) of succinic anhydride in 40 ml of anhydrous o-xylene containing 0.8 mmol of A27Br resin was placed under dry nitrogen atmosphere in a 100 ml glass vial. The vial was sealed and then kept at 70°C for 40 h. The resulting solution was filtered and the solvent removed under vacuum to yield 2.0 g (conv. 43%) of polymeric product that was characterized by GPC, IR and NMR analysis. An equimolar amount of both monomeric units was detected in the copolymer by 1H-NMR.

Example 13 Copolymer of 1-0-glycidyl-2.3-0-isopropylideneglycerol with succinic anhydride.

A solution of 3.0 g (16 mmol) of GIG and 1.6 g (16 mmol) of succinic anhydride in 40 ml of anhydrous toluene containing 0.8 mmol of triisobutylaluminum was placed under dry nitrogen atmosphere in a 100 ml glass vial. The vial was sealed and then kept at 80°C for 48 h. The resulting solution was filtered and the solvent removed under vacuum to yield 4.5 g (conv. 98%) of polymeric product that was characterized by IR and NMR spectroscopy. An equimolar amount of both monomeric units was detected in the copolymer by 1H-NMR.

Example 14 Copolymer of 1-0-glycidyl-2.3-0-isopropylideneglycerol with succinic anhydride

A solution of 3.0 g (16 mmol) of GIG and 1.6 g (16 mmol) of succinic anhydride in 40 ml of anhydrous toluene containing 0.8 mmol of hexaisobutyltetraalumoxane was placed under dry nitrogen atmosphere in a 100 ml glass vial. The vial was sealed and then kept at 80°C for 48 h. The resulting solution was filtered and the solvent removed under vacuum to yield 4.5 g (conv. 98%) of polymeric product that was characterized by GPC, IR and NMR analysis. An equimolar amount of both monomeric units was detected in the copolymer by 1H-NMR.

Example 15 Copolymer of 0-glycidyl-di-0-isopropylidenexylitol with succinic anhydride.

A solution of 4.6 g (16 mmol) of XPG and 1.6 g (16 mmol) of succinic anhydride in 35 ml of anhydrous toluene containing 0.8 mmol of triisobutylaluminum was placed under dry nitrogen atmosphere in a 100 ml glass vial. The vial was sealed and then kept at 80°C for 62 h. The resulting solution was filtered and the solvent removed under vacuum to yield 5.9 g (conv. 96%) of polymeric product that was characterized by GPC, IR and NMR analysis. An equimolar amount of both monomeric units was detected in the copolymer by 1H-NMR.

IR (film): $\bar{\nu}$ = 3000-2800 ($\nu$ CH aliphatic), 1740 ($\nu$ C=O), 1458 ($\delta$ $CH_2$ and $\delta_{as}$ $CH_3$). 1376 ($\delta_s$ $CH_3$) and 1300-1000 $cm^{-1}$ ($\nu$ C-O-C).

$^1$H-NMR ($CDCl_3$): $\delta$ = 1.2-1.4 (m, 12H, $CH_3$), 2.6 (s, 4H, $CH_2$ AS), 3.2-4.4 (m, 11H, CH-O + $CH_2$-O + $CH_2$-OOC) and 5.1 ppm (s, 1H, CH-OOC).

$^{13}$C-NMR ($CDCl_3$): $\delta$ = 25-27 ($CH_3$), 28-29 ($CH_2$ AS), 62-82 (CH-O + $CH_2$-O), 109-110 (O-C-O) and 171.5-172.5 ppm (C=O).

Example 16 Copolymer of L-0-glycidyl-di-0-isopropylidenearabitol (APG) with succinic anhydride.

A solution of 4.6 g (16 mmol) of APG and 1.6 g (16 mmol) of succinic anhydride in 35 ml of anhydrous toluene containing 0.8 mmol of triisobutylaluminum was placed under dry nitrogen atmosphere in a 100 ml glass vial. The vial was sealed and then kept at 80°C for 62 h. The resulting solution was filtered and the solvent removed under vacuum to yield 6.0 g (conv. 97%) of polymeric product that was characterized by GPC, IR and NMR analysis. An equimolar amount of both monomeric units was detected in the copolymer by 1H-NMR.

IR (film): $\bar{\nu}$ = 3000-2800 ($\nu$ CH aliphatic), 1740 ($\nu$ C=O), 1458 ($\delta$ $CH_2$ and $\delta_{as}$ $CH_3$), 1376 ($\delta_s$ $CH_3$) and 1300-1000 $cm^{-1}$ ($\nu$ C-O-C).

$^1$H-NMR ($CDCl_3$): $\delta$ = 1.2-1.4 (m, 12H, $CH_3$), 2.6 (s, 4H, $CH_2$ AS), 3.2-4.4 (m, 11H. CH-O + $CH_2$-O + $CH_2$-OOC) and 5.1 ppm (s, 1H, CH-OOC).

$^{13}$C-NMR ($CDCl_3$): $\delta$ = 24-27 ($CH_3$), 28-29 ($CH_2$ AS), 62-82 (CH-O + $CH_2$-O), 109-110 (O-C-O) and 171.5-172.5 ppm (C=O).

Example 17 Copolymer of N-glycidylpyrrolidone with succinic anhydride.

A solution of 2.0 g (16 mmol) of NPG and 1.6 g (16 mmol) of succinic anhydride in 35 ml of anhydrous toluene containing 0.8 mmol of triisobutylaluminum was placed under dry nitrogen atmosphere in a 100 ml glass vial. The vial was sealed and then kept at 80C for 62 h. The resulting mixture was dissolved in chloroform and poured into excess diethyl ether. The coagulated polymer was dried under vacuum to yield 3.4 g (conv. 95%) of polymeric product that was characterized by GPC, IR and NMR analysis. An equimolar amount of both monomeric units was detected in the copolymer by 1H-NMR.

IR (film): $\bar{\nu}$ = 3000-2800 ($\nu$ CH aliphatic), 1738 ($\nu$ C=O ester), 1688 ($\nu$ C=O lactame), 1464 ($\delta$ $CH_2$ and $\delta_{as}$ $CH_3$), 1378 ($\delta_s CH_3$) and 1300-1000 $cm^{-1}$ ($\nu$ C-O-C and $\nu$ C-N).

$^1$H-NMR ($CDCl_3$): $\delta$ = 2.0 (m, 2H, $CH_2$), 2.3 (t, 2H, $CH_2$-C=O), 2.6 (s, 4H, $CH_2$ AS), 3.0-3.9(m, 4H, $CH_2$-N), 4.0-4.3 (m, 2H, $CH_2$-O) and 5.2 ppm (m, 1H, CH-O).

$^{13}$C-NMR ($CDCl_3$): $\delta$ = 17.76 (C-9), 28.45 (C-2 + C-3), 30.24 (C-10), 42.76 (C-8), 47.89 (C-7 ), 63.10 (C-5), 69.29 (C-6), 171.25 and 171.92 (C-1 + C-4) and 175.95 ppm (C-11).

Example 18 Terpolymer of 1-0-glycidyl-2.3-0-isopropyli deneglycerol with propylene oxide (PO) and carbon dioxide.

A solution of 1.7 g (29 mmol) of PO and 5.9 g (31.3 mmol) of GIG in 21 ml (1.2 mmol) of the DBZ/Pg dioxane solution was placed under dry nitrogen atmosphere in a 150 ml stainless steel pressure vessel that was filled with carbon dioxide up to 40 atm. The polymerization mixture was kept under stirring for 52 h at 35°C. The orange solid obtained was dissolved in chloroform, washed with 5% $NH_4Cl$, water and water in that order and finally poured into a large excess of methanol. The coagulated polymer was dried under vacuum to yield 1.2 g (conv. 12%) of polymeric product that was characterized by GPC, IR and NMR analysis. A 40% molar content of GIG units was detected in the copolymer by 1H-NMR.

IR (film): $\bar{\nu}$ = 3000-2800 ($\nu$ CH aliphatic), 1748 ($\nu$ C=O), 1456 ($\delta$ $CH_2$ and $\delta_{as}$ $CH_3$), 1380 ($\delta_s$ $CH_3$) and 1300-1000 $cm^{-1}$ ($\nu$ C-O-C).

$^1$H-NMR ($CDCl_3$): $\delta$ = 1.2-1.5 ($CH_3$), 3.5 and 3.7 (5H GIG), 4.2 (2H PO and 4H GIG) and 5.0 ppm (1H GIG and 1H PO).

$^{13}$C-NMR ($CDCl_3$): $\delta$ = 16.03 ($CH_3$ PO), 25.24 and 26.57 ($CH_3$ GIG), 65.5-75 (CH-O + $CH_2$-O), 109.52 (O-C-O) and 153.5-155.5 ppm (C=O).

Example 19 Terpolymer of 1-0-glycidyl-2.3-0-isopropylideneglycerol with propylene oxide and carbon dioxide.

A solution of 8.7 g (150 mmol) of PO and 14.5 g (77 mmol) of GIG in 21 ml (5.8 mmol) of the DBZ/Pg dioxane solution was placed under dry nitrogen atmosphere in a 150 ml stainless steel pressure vessel that was filled with carbon dioxide up to 40 atm. The polymerization mixture was kept under stirring for 85 h at 35°C. The orange solid obtained was dissolved in chloroform, washed with 5% $NH_4Cl$, water and water in that order and finally poured into a large excess of methanol. The coagulated polymer was dried under vacuum to yield 6.2 g (conv. 19%) of polymeric product that was characterized by GPC, IR and NMR analysis. A 25% molar content of GIG units was detected in the copolymer by 1H-NMR.

Example 20 Terpolymer of 1-O-glycidyl-2.3-O-isopropylideneglycerol with propylene oxide and carbon dioxide.

A solution of 7.0 g (120 mmol) of PO and 5.2 g (27.4 mmol) of GIG in 21 ml (5.8 mmol) of the DBZ/Pg dioxane solution was placed under dry nitrogen atmosphere in a 150 ml stainless steel pressure vessel that was filled with carbon dioxide up to 40 atm. The polymerization mixture was kept under stirring for 64 h at 35°C. The orange solid obtained was dissolved in chloroform, washed with 5% $NH_4Cl$, water and water in that order and finally poured into a large excess of methanol. The coagulated polymer was dried under vacuum to yield 3.3 g (conv. 17%) of polymeric product that was characterized by GPC, IR and NMR analysis. A 15% molar content of GIG units was detected in the copolymer by 1H-NMR.

Example 21 Terpolymer of 0-glycidyl-di-0-isopropylidenexylitol with propylene oxide and carbon dioxide

A solution of 2.3 g (40 mmol) of PO and 3.4 g (12 mmol) of XPG in 7.5 ml (1.8 mmol) of the DBZ/Pg dioxane solution was placed under dry nitrogen atmosphere in a 150 ml stainless steel pressure vessel that was filled with carbon dioxide up to 40 atm. The polymerization mixture was kept under stirring for 90 h at 35°C. The orange solid obtained was dissolved in chloroform. washed with 5% $NH_4Cl$, water and water in that order and finally poured into a large excess of methanol. The coagulated polymer was dried under vacuum to yield 1.4 g (conv. 18%) of polymeric product that was characterized by GPC, IR and NMR analysis. A 7% molar content of XPG units was detected in the copolymer by 1H-NMR.

IR (film): $\bar{\nu}$ = 3000-2800 ($\nu$ CH aliphatic), 1746 ($\nu$ C=O), 1458($\delta$ $CH_2$ and $\delta_{as}$ $CH_3$), 1380 ($\delta_s$ $CH_3$) and 1300-1000 $cm^{-1}$ ($\nu$ C-O-C).
$^1$H-NMR ($CDCl_3$):$\delta$ = 1.2-1.4 ($CH_3$), 3.4-4.4 (11H XPG and 2H PO) and 5.0 ppm (1H XPG and 1H PO).
$^{13}$C-NMR ($CDCl_3$): $\delta$ = 15,93 ($CH_3$ PO), 25-27 ($CH_3$ XPG), 65.5-81.3

Example 22 Terpolymer of 0-glycidyl-di-0-isopropylidenexylitol with propylene oxide and carbon dioxide.

A solution of 1.5 g (26 mmol) of PO and 7.7 g (27 mmol) of XPG in 7.5 ml (1.8 mmol) of the DBZ/Pg dioxane solution was placed under dry nitrogen atmosphere in a 150 ml stainless steel pressure vessel that was filled with carbon dioxide up to 40 atm. The polymerization mixture was kept under stirring for 90 h at 35°C. The orange solid obtained was dissolved in chloroform, washed with 5% $NH_4Cl$, water and water in that order and finally poured into a large excess of methanol. The coagulated polymer was dried under vacuum to yield 0.8 g (conv. 7%) of polymeric product that was characterized by GPC, IR and NMR analysis. A 8% molar content of XPG units was detected in the copolymer by 1H-NMR.

Example 23 Terpolymer of 0-glycidyl-di-0-isopropylidenexylitol with propylene oxide and carbon dioxide.

A solution of 1.4 g (24 mmol) of PO and 7.0 g (24 mmol) of XPG in 7.5 ml (1.8 mmol) of the DBZ/Pg dioxane solution was placed under dry nitrogen atmosphere in a 150 ml stainless steel pressure vessel that was filled with carbon dioxide up to 40 atm. The polymerization mixture was kept under stirring for 95 h at 35°C. The orange solid obtained was dissolved in chloroform, washed with 5% $NH_4Cl$, water and water in that order and finally poured into a large excess of methanol. The coagulated polymer was dried under vacuum to yield 0.8 g (conv. 8%) of polymeric product that was characterized by GPC, IR and NMR analysis. A 7% molar content of XPG units was detected in the copolymer by 1H-NMR.

Reaction of deprotection

Example 24 Copolymer of 1-0-glycidylglycerol with succinic anhydride.

A solution of 2.5 g of GIG/SA copolymer in a mixture of 80 ml of methanol and 100 ml of chloroform was placed under dry nitrogen in a 250 ml two necked flask equipped with condenser and magnetic stirring. After addition of 2.5 g of Amberlyst 15, the mixture was kept under stirring at 45°C for 64 h, then the resin was filtered off and the volatile products removed under vacuum to yield 1.9 g of a semisolid product that was characterized by GPC, IR and NMR analysis.

IR (film): $\bar{\nu}$ = 3600-3100 ($\nu$ O-H), 3000-2800 ($\nu$ CH aliphatic), 1740 ($\nu$ C=O), 1464 ($\delta$ $CH_2$ and $\delta_{as}$ $CH_3$), 1378 ($\delta_s$ $CH_3$), 1300-1000 ($\nu$ C-O-C) and 800-400 $cm^{-1}$ ($\delta$ O-H).
$^1$H-NMR ($CDCl_3$): $\delta$ = 2.7 (m, $CH_2$ AS), 3.5-4.4 (m, CH-O + $CH_2$-O) and 4.9 ppm (s, OH).
$^{13}$C-NMR ($CDCl_3$): $\delta$ = 25.09 and 26.43 ($CH_3$), 29.5-30.5 ($CH_2$ AS), 52-75.5 (CH-O + $CH_2$-O) and 173.5-175 ppm (C=O).

Example 25 Copolymer of 0-glycidylxylitol with succinic anhydride.

A solution of 2.5 g of XPG/SA copolymer in a mixture of 80 ml of methanol and 80 ml of chloroform was placed under dry nitrogen in a 290 ml two necked flask equipped with condenser and magnetic stirring. After addition of 2.5 g of Amberlyst 15, the mixture was kept under stirring at 45°C for 64 h, then the resin was filtered off and the volatile products removed under vacuum to yield 1.9 g of a semisolid product that was characterized by GPC, IR and NMR analysis.

IR (film): $\bar{\nu}$ = 3600-3100 ($\nu$ O-H), 3000-2800 ($\nu$ CH aliphatic), 1740 ($\nu$ C=O), 1464 ($\delta$ $CH_2$ and $\delta_{as}$, $CH_3$), 1378 ($\delta_s$ $CH_3$), 1300-1000 ($\nu$ C-O-C) and 800-400 $cm^{-1}$ ($\delta$ O-H).
$^1$H-NMR ($CDCl_3$): $\delta$ = 2.7 (m, $CH_2$AS), 3.5-4.4 (m, CH-O+$CH_2$-O) and 4.9 ppm (s, OH).
$^{13}$C-NMR ($CD_3OD$): $\delta$ =29-30.5 ($CH_2$ AS), 60-83 (CH-O+$CH_2$-O) and 173-175 ppm (C=O).

Example 26 Copolymer of 0-glycidylarabitol with succinic anhydride.

A solution of 2.0 g of APG/SA copolymer in a mixture of 70 ml of methanol and 70 ml of chloroform was placed under dry nitrogen in a 250 ml two necked flask equipped with condenser and magnetic stirring. After addition of 2.0 g of Amberlyst 15, the mixture was kept under stirring at 45C for 64 h, then the resin was filtered off and the volatile products removed under vacuum to yield 1.5 g of a semisolid product that was characterized by GPC, IR and NMR analysis.

IR (film): $\bar{\nu}$= 3600-3100 ($\nu$ O-H), 3000-2800 ($\nu$ CH aliphatic), 1740 ($\nu$ C=O), 1464 ($\delta$ $CH_2$ and $\delta_{as}$ $CH_3$), 1378 ($\delta_s$ $CH_3$), 1300-1000 ($\nu$ C-O-C) and 800-400 $cm^{-1}$ ($\delta$ O-H).
$^1$H-NMR ($CDCl_3$): $\delta$ = 2.7 (m, $CH_2$ AS), 3.5-4.4 (m, CH-O + $CH_2$-O) and 4.9 ppm (s, OH).
$^{13}$C-NMR ($CD_3OD$): $\delta$=29-30.5 ($CH_2$ AS), 61-82 (CH-O+$CH_2$-O) and 173-175 ppm (C=O).

Example 27 Terpolymer of 1-0-glycidylglycerol with propylene oxide and carbon dioxide.

A solution of 2.0 g of GIG/PO/CO2 terpolymer containing 15%-mol of GIG units in 70 ml of dichloromethane was poured under vigorous stirring in 300 ml of 20:1 methanol/concentrated HCl mixture and left under stirring for 2 h. The coagulated polymer was then washed with methanol, dissolved in chloroform, precipitated in diethyl ether and finally dried under vacuum to yield 2.9 g of a white powder that was characterized by GPC, IR and NMR analysis.

IR (film): $\bar{\nu}$ = 3600-3100 ($\nu$ O-H), 3000-2800 ($\nu$ CH aliphatic), 1740 ($\nu$ C=O), 1464 ($\delta$ $CH_2$ and $\delta_{as}$ $CH_3$), 1378 ($\delta_s$ $CH_3$), 1300-1000 ($\nu$ C-O-C) and 800-400 $cm^{-1}$ ($\delta$ O-H).
$^1$H-NMR ($CDCl_3$): $\delta$ = 1.2-1.5 ($CH_3$), 3.3-4.3 ($CH_2$-O + CH-O) and 4.9 ppm (CH-OOC).
$^{13}$C-NMR ($CDCl_3$): $\delta$ = 15.96 ($CH_3$ PO), 63-75 (CH-O+$CH_2$-O) and 153.5-155 ppm (C=O).

Example 28 Terpolymer of 1-0-glycidylglycerol with propylene oxide and carbon dioxide.

A solution of 4.8 g of GIG/PO/$CO_2$ terpolymer containing 25%-mol of GIG units in 50 ml of dichloromethane was poured under vigorous stirring in 400 ml of 20:1 methanol/concentrated HCl mixture and left under stirring for 2 h. The coagulated polymer was then washed with methanol, dissolved in chloroform, precipitated in diethyl ether and finally dried under vacuum to yield 3.7 g of a white powder that was characterized by GPC, IR and NMR analysis.

Example 29 <u>Terpolymer of 1-0-glycidylglycerol with propylene oxide and carbon dioxide</u>.

A solution of 0.8 g of GIG/PO/$CO_2$ terpolymer containing 40%-mol of GIG units in 20 ml of dichloromethane was poured under vigorous stirring in 100 ml of 20:1 methanol/concentrated HCl mixture and left under stirring for 2 h. The coagulated polymer was then washed with methanol, dissolved in chloroform, precipitated in diethyl ether and finally dried under vacuum to yield 0.4 g of a white powder that was characterized by GPC. IR and NMR analysis.

Example 30 <u>Terpolymer of 0-glycidylxylitol with propylene oxide and carbon dioxide</u>.

A solution of 0.8 g of XPG/PO/$CO_2$ terpolymer containing 7% mol of XPG units in 29 ml of chloroform was poured under vigorous stirring in 200 ml of 50:1 methanol/concentrated HCl mixture and left under stirring for 2 h. The coagulated polymer was then washed with methanol, dissolved in chloroform, precipitated in diethyl ether and finally dried under vacuum to yield 0.6 g of a white powder that was characterized by GPC, IR and NMR analysis.

IR (film): $\bar{\nu}$ = 3600-3100 ($\nu$ O-H), 3000-2800 ($\nu$ CH aliphatic), 1740 ($\nu$ C=O), 1464 ($\delta$ $CH_2$ and $\delta_{as}$ $CH_3$), 1378 ($\delta$, $CH_3$), 1300-1000 ($\nu$ C-O-C) and 800-400 $cm^{-1}$ ($\delta$ O-H).
$^1$H-NMR ($CDCl_3$): $\delta$ = 1.2-1.5 ($CH_3$), 3.4-4.4 ($CH_2$-O + CH-O) and 5.0 ppm (CH-OOC).
$^{13}$C-NMR ($CDCl_3$): $\delta$ = 15.93 ($CH_3$ PO), 25-27 ($CH_3$ XPG), 65.5-81.5 (CH-O + $CH_2$-O), 109-110 (O-C-O) and 153.5-155 ppm (C=O).

Example 31 <u>Terpolymer of 0-glycidylxylitol with propylene oxide and carbon dioxide</u>.

A solution of 0.5 g of XPG/PO/$CO_2$ terpolymer containing 8%-mol of XPG units in 20 ml of chloroform was poured under vigorous stirring in 150 ml of 50:1 methanol/concentrated HCl mixture and left under stirring for 2 h. The coagulated polymer was then washed with methanol, dissolved in chloroform, precipitated in diethyl ether and finally dried under vacuum to yield 0.3 g of a white powder that was characterized by GPC, IR and NMR analysis.

Example 32 <u>Terpolymer of 0-glycidylxylitol with propylene oxide and carbon dioxide</u>.

A solution of 0.3 g of XPG/PO/$CO_2$ terpolymer containing 11%-mol of XPG units in a mixture of 10 ml of methanol and 20 ml of chloroform was placed under dry nitrogen in a 100 ml two necked flask equipped with condenser and magnetic stirring. After addition of 0.2 g of Amberlyst 15, the mixture was kept under stirring at 45°C for 65 h, then the resin was filtered off and the volatile products removed under vacuum to yield 0.2 g of a semisolid product that was characterized by GPC, IR and NMR analysis.

While certain specific considerations have been disclosed and discussed herein, such have been offered solely to exemplify the present invention, and should in no way be considered as limiting the scope and nature of the invention.

## Claims

1. Biodegradable compounds having the following formula

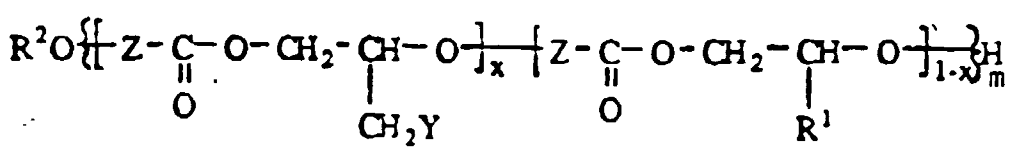

where:

- R1 is either H, or alkyl, or 1-(2-pyrrolidonyl) alkoxymethyl, or 1-(2- pyrrolidonyl)alkyl;
- R2 is either H, or alkyl, or aryl;
- Z is either -CO-R3- residue where R3 is linear or branched alkanediyl or arylalkanediyl or arylenediyl, or is substituted by a carbon-oxygen bond;
- Y is either glyceryl, or pentitolyl, or heptitolyl;
- O < x ≤ 1; 5 < m < 500.

2. Biodegradable compounds according to claim 1 in which Z is a CO-R3 residue where R3 is a linear o branched

alkanediyl or arylalkanediyl or arylenediyl.

3. Biodegeadable compounds according to claim 1 in which Z is substituted by a carbon-oxygen bond.

4. Biodegradable compounds according to any of the previous claims in which R1 is a methyl.

5. Biodegradable compounds according to any of the previous claims characterized by having a molecular weight between 1000 and 100.000.

6. Biodegradable compounds according to claim 5 characterized by having a molecular weight between 2.000 and 30.000.

7. Biodegradable compounds according to any of the previous claims in which $10 < m < 150$.

8. Biodegradable compounds having the formula of claim 1 characterized in that they derive by copolymerization of the glycidyl ethers of alditols protected with isopropylidene groups and their mixtures with alkylene oxides with anhydrides, either cyclic anhydrides or carbon dioxide, in the presence of catalysts; and by controlled removal of said isopropylidene groups.

9. Biodegradable compounds according to claim 8 in which said anhydrides are cyclic anhydrides.

10. Biodegradable compounds according to claim 9 in which said cyclic anhydride is succinic anhydride.

11. Biodegradable compounds according to claim 9 or 10 in which said copolymerization is at a temperature between 30C and 150C and for a time comprised between 2 and 120 hrs.

12. Biodegradable compounds according to any of the claims from 9 to 11 in which said catalysts is a alkylaluminum derivative.

13. Biodegradable compounds according to claim 8 in which said anhydride is carbon dioxide.

14. Biodegradable compounds according to claim 13 in which said catalysts is the dialkylzinc/polyhydroxyphenols system.

15. Biodegradable compounds according to any of the claims from 8 to 14 in which said glycidyl ethers of alditols have either three, or five, or seven carbon atoms and said alkylene oxides are either ethylene oxide or propylene oxide.

16. Biodegradable compounds according to claim 15 in which said glycidyl ethers of alditols is chosen in the following groups:

1-0-glycidyl-2,3-0-isopropylideneglycerol,
0-glycidyl-di-0-isopropylidenexylitol,
1-0-glycidyl-di-0-isopropylidenearabitol,
N-glycidylpyrrolidone.

17. Process for the production of a biodegradable compound having the following formula

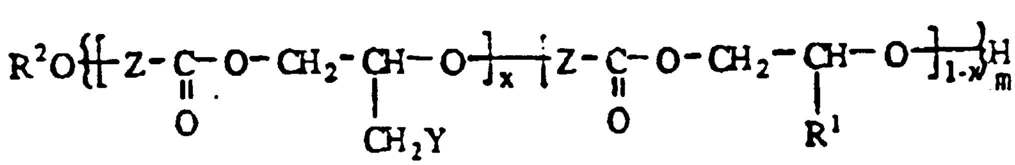

where:

- R1 is either H, or alkyl, or 1-(2-pyrrolidonyl) alkoxymethyl, or 1-(2- pyrrolidonyl)alkyl;
- R2 is either H, or alkyl, or aryl;

- Z is either -CO-R3- residue where R3 is linear or branched alkanediyl or arylalkanediyl or arylenediyl, or is substituted by a carbon-oxygen bond;
- Y is either glyceryl, or pentitolyl, or heptitolyl;
- $O < x \leq 1$; $5 < m < 500$

characterized by a copolymerization of the glycidyl ethers of alditols protected with isopropylidene groups and their mixtures with alkylene oxides with anhydrides, either cyclic anhydrides or carbon dioxide, in the presence of catalysts; and by controlled removal of said isopropylidene groups.

**18.** Process according to claim 17 in which said anhydrides are a cyclic anhydrides.

**19.** Process according to claim 18 in which said cyclic anhydride is succinic anhydride.

**20.** Process according to claim 17 to 19 in which said copolymerization is at a temperature between 30C and 150C and for a time comprised between 2 and 120 hrs.

**21.** Process according to any of claims from 17 to 20 in which said catalysts is a alkylaluminum derivative.

**22.** Process according to claim 17 in which said anhydride is carbon dioxide.

**23.** Process according to claim 22 in which said copolymerization is at a temperature between 30C and 60C and for a time comprised between 20 and 120 hrs.

**24.** Process according to claim 22 or 23 in which said catalysts is the dialkylzinc/polyhydroxyphenols system.

**25.** Process according to any of claims from 17 to 24 in which said glycidyl ethers of alditols have either three, or five, or seven carbon atoms and said alkylene oxides are either ethylene oxide or propylene oxide.

**26.** Process according to claim 25 in which said glycidyl ethers of alditols is chosen in the following groups: 1-0-glycidil-2,3-0-isopropylideneglycerol, 0-glycidyl-di-0-isopropylidenexylitol, 1-0-glycidyl-di-0-isopropylidenearabitol, N-glycidylpyrrolidone.

**27.** Process according to any of claims from 17 to 26 in which said removal of isopropylidene groups is carried out by precipitation in ethanol with 5% concentrated HCl, for a time comprised between 30' and 5 hrs.

**28.** Process according to any of claims from 17 to 26 in which said removal is carried out by heating at a temperature between 40C and 50C the polymer solution in methanol/chloroform mixtures in the presence of a styrene/divinylbenzene sulphonic resin for a time comprised between 40 and 80 hrs.

**Patentansprüche**

**1.** Biologisch abbaubare Verbindungen der folgenden Formel:

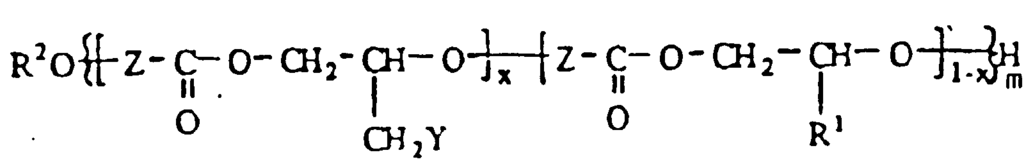

worin,

- $R^1$ ist entweder H, oder Alkyl, oder 1-(2-Pyrrolidonyl)-Alkoxymethyl, oder 1-(2-Pyrrolidonyl)Alkyl;
- $R^2$ ist H oder Alkyl, oder Aryl;
- Z ist ein -CO-$R^3$-Rest, worin $R^3$ ein entweder lineares oder entweder abgezweigtes Alkanediyl oder Arylalkanediyl oder Arylenediyl ist oder durch eine Kohlenstoff-Sauerstoff-Bindung ersetzt ist;
- Y ist Glyceryl oder Pentitolyl, oder Heptitolyl;

- $O < x \leq 1$; $5 < m < 500$.

**2.** Biologisch abbaubare Verbindungen nach Anspruch 1, worin Z ein CO-$R^3$-Rest ist, wobei $R^3$ ein lineares oder abgezweigtes Alkanediyl oder Arylalkanediyl oder Arylenediyl darstellt.

**3.** Biologisch abbaubare Verbindungen nach Anspruch 1, worin Z durch eine Kohlenstoff-Sauerstoff-Bindung ersetzt ist.

**4.** Biologisch abbaubare Verbindungen nach je einem der vorhergehenden Ansprüche, worin $R_1$ ein Methyl ist.

**5.** Biologisch abbaubare Verbindungen nach je einem der vorhergehenden Ansprüche, dadurch gekennzeichnet, dass sie ein Molekulargewicht zwischen 1.000 und 100.000 aufweisen.

**6.** Biologisch abbaubare Verbindungen nach Anspruch 5, dadurch gekennzeichnet, dass sie ein Molekulargewicht zwischen 2.000 und 30.000 aufweisen.

**7.** Biologisch abbaubare Verbindungen nach je einem der vorhergehenden Ansprüche, worin $10 < m < 150$ ist.

**8.** Biologisch abbaubare Verbindungen mit der im Anspruch 1 angegebenen Formel, dadurch gekenzeichnet, dass sie durch Kopolymerisation von den Glycidyl-Äthern von Alditolen, geschützt mit Isopropyliden-Gruppen und deren Mischungen mit Alkylenoxiden, mit Anhydriden, entweder zyklischen Anhydryden oder Kohlendioxyden, in Gegenwart von Katalysatoren, und durch eine kontrolierte Abführung der vorgenannten Isopropylidene-Gruppen erhalten werden.

**9.** Biologisch abbaubare Verbindungen nach Anspruch 8, worin die vorgenannten Anhydride zyklische Anhydride sind.

**10.** Biologisch abbaubare Verbindungen nach Anspruch 9, worin das vorgenannte zyklische Anhydrid das Bernsteinanhydrid ist.

**11.** Biologisch abbaubare Verbindungen nach Anspruch 9 oder 10, worin die vorgenannte Kopolymerisation bei einer Temperatur zwischen 30° und 150°C und in einer zeitdauer zwischen 2 und 120 stunden durchgeführt wird.

**12.** Biologisch abbaubare Verbindungen nach je einem der Ansprüche 9 bis 11, worin der vorgenannte Katalysator ein Alkylaluminumderivat ist.

**13.** Biologisch abbaubare Verbindungen nach Anspruch 8, worin das vorgennante Anhydrid Kohlendioxyd ist.

**14.** Biologisch abbaubare Verbindungen nach Anspruch 13, worin der vorgenannte Katalysator ein Dialkylzink/Polyhydroxyphenol-System ist.

**15.** Biologisch abbaubare Verbindungen nach je einem der Ansprüche 8 bis 14, worin die vorgenannten Glycidyl-Aether von Alditolen drei oder fünf oder sieben Kohlenstoffatome aufweisen und die vorgenannten Alkylen-Oxyde entweder Aethylen- oder Propylenoxid sind.

**16.** Biologisch abbaubare Verbindungen nach Anspruch 15, worin die vorgenannten Glycidyl-Aether von Alditolen von den folgenden Gruppen ausgewählt sind:

1-O-Glycidyl-2,3-O-Isopropylidenglycerol,
O-Glycidyl-di-O-Isopropylidenxylitol,
1-O-Glycidyl-di-O-Isopropylidenarabitol,
N-Glycidylpyrrolidone.

**17.** Verfahren zur Herstellung einer biologisch abbaubaren Verbindung der folgenden Formel:

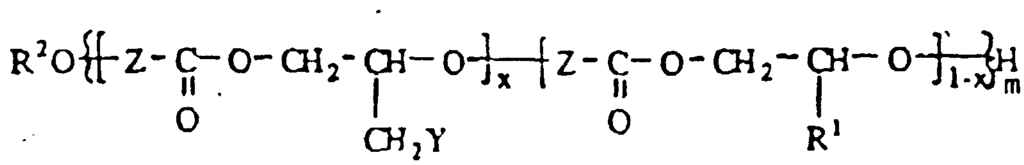

worin:

- $R^1$ entweder H oder Alkyl oder 1-(2-Pyrrolidonyl) Alkoxymethyl oder 1-(2-Pyrrolidonyl) Alkyl ist;
- $R^2$ entweder H oder Alkyl oder Aryl ist;
- Z entweder ein -CO-$R^3$-Rest, worin $R^3$ ein lineares oder abgezwligtes Alkandiyl oder Arylalkandiyl oder Arylendiyl ist oder durch eine Kohlenstoff-Sauerstoff-Bindung ersetzt ist;
- Y entweder Glyceryl oder Pentitolyl oder Heptitolyl ist;
- $O < m \leq 1$; $5 < m < 500$;

gekennzeichnet durch eine Kopolymerisation der Glycidyl-Aethern von Alditolen, geschützt mit Isopropylengruppen und deren Mischungen mit Alkyleoxiden, mit Anhydriden, entweder zyklischen Anhydriden oder Kohlendioxyd, in Gegenwart von Katalysatoren, und durch eine kontrollierte Abführung der vorgenannten Isopropylidengruppen.

**18.** Verfahren nach Anspruch 17, worin die vorgenannten Anhydriden ein zyklisches Anhydrid sind.

**19.** Verfahren nach Anspruch 18, worin die vorgenannten Anhydriden ein Bernsteinanhydrid sind.

**20.** Verfahren nach den Ansprüchen 17 bis 19, worin die vorgenannte Kopolymerisation bei einer Temperatur zwischen 30° und 150°C und für eine Zeitdauer zwischen 2 und 120 Studen durchgeführt wird.

**21.** Verfahren nach den Ansprüchen 17 bis 19, worin der vorgenannte Katalysator ein Alkylaluminiumderivat ist.

**22.** Verfahren nach Anspruch 17, worin das vorgenannte Anhydrid Kohlendioxid ist.

**23.** Verfahren nach Anspruch 22, worin die vorgenannte Kopolymerisation bei einer Temperatur zwischen 30°C und 60°C und für eine Zeitdauer zwischen 20 und 120 Studen durchgeführt wird.

**24.** Verfahren nach Anspruch 22 oder 23, worin der vorgenannte Katalysator ein Dialkylzink/Polyhydroxyphenol-System ist.

**25.** Verfahren nach je einem der Ansprüche 17 bis 24, worin die vorgenannten Glycidyl-Aethern von Alditolen entweder drei oder fünf oder sieben Kohlenstoffatome aufweisen und die vorgenannten Alkylenoxide entweder Aethylen- oder Propylen-Oxide sind.

**26.** Verfahren nach Anspruch 25, worin die vorgenannten Glycidyl-Aethern von Alditolen von den folgenden Gruppen ausgewählt werden:

1-O-Glycidyl-2,3-O-Isopropylidenglycerol,
O-Glycidyl-di-O-Isopropylidenxylitol,
1-O-Glycidyl-di-O-Isopropylidenarabitol,
N-Glycidylpyrrolidone.

**27.** Verfahren nach je einem der Ansprüche 17 bis 26, worin die vorgenannte Abführung von Isopropylidengruppen durch Niederschlagung in Aethanol mit 5% von konzentrierten HCl für einer Zeitdauer zwischen 30 Minuten und 5 Stunden durchgeführt wird.

**28.** Verfahren nach je einem der Ansprüche 17 bis 26, worin die vorgenannte Abführung durch Erwärmung auf eine Temperatur zwischen 40°C und 50°C einer Polymerlösung in einer Methanol/Chloroform-Mischung in Gegenwart eines Styren/Divinylbenzol-Sulfonharzes für eine Zeitdauer zwischen 40 und 80 Stunden durchgeführt wird.

## Revendications

**1.** Composés biodégradables ayants la formule suivante:

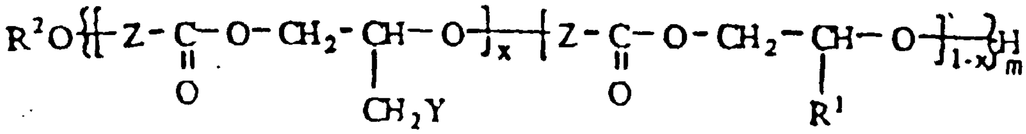

dans laquelle:

- $R_1$ est ou H, ou Alcoyle, ou 1-(2-pyrrodinoyl) alcoxyméthyle, ou 1-(2-pyrrolidonyl)alcoyle;
- $R_2$ est ou H, ou alcoyle ou aryle;
- Z est ou residu de -CO-$R_3$, dans lequel $R_3$ est alcanediyle ou arylalcandiyle ou arylcanediyle ou arylenediyle, lineaire ou remifié ou est substitué par une liaison carbone-oxygène;
- Y est ou glyceryle ou pentitolyle ou heptitolyle;
- $0 < x \leq 1$; $5 < m < 500$

**2.** Composés biodégradables selon la revendication 1, dans lesquels Z est un résidu de CO-$R_3$, dans lequel $R_3$ est un alcanediyle ou arylalcanediyle ou arilendiyle lineaire ou ramifié.

**3.** Composés biodégradables selon la revendication 1, dans lesquels Z est substitué par une liaison carbone-oxygène.

**4.** Composés biodégradables selon une quelconque des revendication précédentes, dans lesquels $R_1$ est méthyle.

**5.** Composés biodégradables selon une quelconque des revendication précédentes, caractérises en ce qu'ils ont un poids moléculaire entre 1.000 et 100.000.

**6.** Composés biodégradables selon la revendication 5, caractérisés en ce qu'ils ont un poids molécular entre 2.000 et 30.000.

**7.** Composés biodégradables selon l'une quelconque des revendications précédentes, dans lesquels $10 < m < 150$.

**8.** Composés biodégradables ayants la formule de la revendication 1, caractérisés en ce qu'ils dérivent de la copolymérisation des éthers glycidyliques des alditols - protegés par des groupes isopropylideniques et leur mélange avec oxides d'alcoylene - avec des anhydrides ou anhydrides cycliques ou dioxide de carbone, en présence de catalyseurs et de la séparation controllé desdits groupes isopropylideniques.

**9.** Composés biodégradables selon la revendication 8, dans lesquels lesdits anhydrides sont des anhydrides cycliques.

**10.** Composés biodégradables selon la revendication 9, dans lesquels ledit anhydride cyclique est l'anhydride succinique.

**11.** Composés biodégradables selon la revendication 9 ou 10, dans lesquels ladit copolymérisation a lieu à une température entre 30 et 150°C et par un temps entre 2 et 120 heures.

**12.** Composés biodégradables selon l'une quelconque des revendications 9 à 11, dans lesquels lesdits catalyseurs sont derivés d'alcoyle-aluminium.

**13.** Composés biodégradables selon la revendication 8, dans lesquels ledit anhydride est le dioxide de carbon.

**14.** Composés biodégradables selon la revendication 13, dans lesquels lesdits catalyseurs sont composé d'un système dialcoylezinc/polyhydroxyphenols.

**15.** Composés biodégradables selon l'une quelconque des revendication 8 à 14, dans lesquels lesdits éthers glycylidiques des alditols ont trois, cinq ou sept atoms de carbone et lesdits oxides d'alcoyilene sont des oxides d'éthylène ou des oxides de propylène.

**16.** Composés biodégradables selon la revendication 15, dans lesquels lesdits éthers glycylidiques des alditols sont choisis dans les groupes suivants:

1-O-glycidyl-2,3-O-isopropylidèneglycérol,
O-glycidyl-di-O-isopropylidènexylitol,
1-O-glycidyl-di-O-isopropylidènearabitol,
N-glycidylpyrrolidone.

**17.** Procéde pour la production d'un composé biodégradable ayant la formule suivante

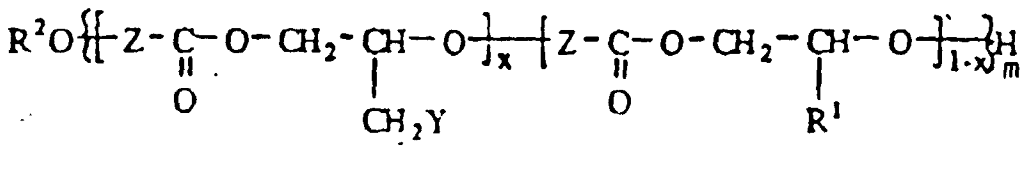

dans lequelle:

- $R_1$ est ou H, ou alcoyle, ou 1-(2-pyrrolidonyl)alcoxyméthyl, ou bien 1-(2-pyrrolidonyl)alcoyle;
  $R_2$ est ou H, ou alcoyle, ou bien aryle;
- Z est ou un résidu de $-CO-R_3$, dans lequel $R_3$ est alcanediyle ou arylealcanediyle ou arylènediyle lineaires ou ramifimiés, ou bien est substitué par une liason carbone-oxigène;
- Y est ou glyceryle, ou pentitolyle, ou heptitolyle;
- O < m< 500;

caractérisé par une copolymérisation des éthers glycidylique des alditols protegés par des gaupes isopropylidèniques et leurs mélanges avec des oxides alcoyilèniques - avec des anhydrides, anhydrides cyclique ou dioxyde de carbon, en présence des catalyseurs; et par une séparation controllée desdits groupes isopropylidèniques.

**18.** Procédé selon la revendication 17, dans lequel lesdits anhydrides sont des anhydrides cycliques.

**19.** Procédé selon la revendication 18, dans lequel ledit anhydride cyclique est l'anhydride succinique.

**20.** Procédé selon les revendications 17 à 19, dans lequel ladite copolymérisation a lieu à une température entre 30°C et 150°C et pour un temps compris entre 2 et 150 heures.

**21.** Procédé selon les revendications 17 à 20, dans lequel ledit catalyseur est un dérivé d'alcoylealuminium.

**22.** Procédé selon la revendication 17, dans lequel ledit anhydride est le dioxide de carbone.

**23.** Procédé selon la revendication 22, dans lequel la dite copolymérisation a lieu à une température entre 30°C et 60°C et pour un temps compris entre 20 et 150 heures.

**24.** Procédé selon les revendications 22 ou 23, dans lequel ledit catalysuer est un système dialcoylezinc/polyhydroxyphenoles.

**25.** Procédé selon l'une quelconque des revendications 17 à 24, dans lequel lesdits éthers glycidyliques des alditoles ont ou trois ou cinq, ou bien sept atomes de carbone et lesdits oxides d'alcoylène sont ou l'oxide d'éthylène ou bien l'oxide de propylène.

**26.** Procédé selon la revendication 25, dans lequel lesdits éthers glycidyliques des alditoles sont choisis dans les groupes suivants:

**27.** Procédé selon l'une quelconque des revendications 17 à 26, dans lequel ladite séparation des groupes isopropylideniques est réalisèe par précipitation en éthanol avec Hcl concentré à 5%, pour un temps compris entre 30' et 5 heures.

**28.** Procédé selon l'une quelconque des revendications 17 à 27, dans lequel ladite séparation des groupes isopropylideniques est réalisée par chauffage à une température comprise entre 40°C et 50°C de la solution de polymère dans mélanges méthanol/chloroforme en présence d'une résine sulphonique de styrène/divinilbenzène pour un temps compris entre 40 et 80 heures.